# EUROPEAN PATENT APPLICATION

(11) **EP 4 541 349 A1**
(43) Date of publication of application: **23.04.2025**
(21) Application number: 23843288.4
(22) Date of filing: 14.07.2023
(51) Int. Cl.: A61K 9/16, A61K 31/58

(54) **SUSTAINED-RELEASE INJECTABLE COMPOSITION CONTAINING DUTASTERIDE**

(30) Priority: 19.07.2022 KR 20220088959; 10.07.2023 KR 20230088892
(71) Applicant: Inventage Lab Inc., Gyeonggi-do 13403 (KR)
(72) Inventor: KIM, Min Sung, Seongnam-si Gyeonggi-do 13494 (KR); KIM, Ju Hee, Seongnam-si Gyeonggi-do 13494 (KR)
(74) Representative: BCKIP Part mbB
(86) International application number: PCT/KR2023/010142
(87) International publication number: WO 2024/019439

(57) **Abstract**

The present invention relates to a sustained-release injectable composition containing dutasteride, which may exhibit a sustained dutasteride release effect for 3 months or more even when the dose of dutasteride is equal to or less than that of existing AVODART^{®}, which is taken once a day, thereby exhibiting a long-acting drug effect by a single injection. In addition, the injectable composition may exhibit a therapeutic effect on benign prostatic hyperplasia, prostate cancer, and hair loss continuously for 3 months or more, may maintain an effective concentration of the drug at a constant level by controlling the release of the drug due to the constant average diameter of the microparticles contained in the sustained-release injectable composition, and may reduce foreign body sensation and pain when administered by injection to a patient.

## Description

### Technical Field

The present invention relates to a sustained-release injectable composition containing dutasteride.

### Background Art

Dutasteride (compound: 17β-N-(2,5-bis(trifluoromethyl))phenylcarbamoyl-4-aza-5α-androst-1-en-3-one), represented by the following Formula 1, is a dual 5-alpha reductase inhibitor that inhibits both types 1 and 2 5-alpha reductase, and is known to be useful for treating benign prostatic hyperplasia, prostate cancer, and androgenetic alopecia by inhibiting the conversion of testosterone to dihydrotestosterone (DHT):

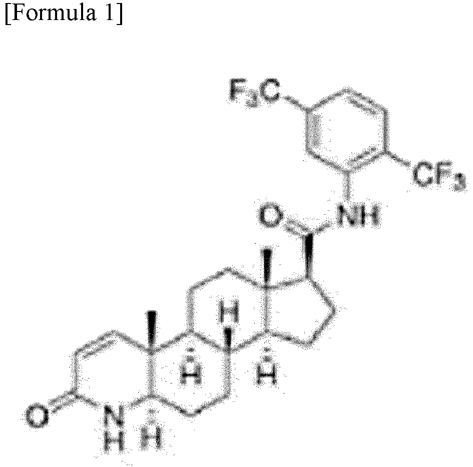

Dutasteride is currently marketed under the trade name AVODART^{®}. AVODART^{®} is a product made by dissolving 0.5 mg of dutasteride in 349.5 mg of a mixture of caprylic/capric mono- and di-glyceride oils and butylated hydroxytoluene (BHT) and then filling a soft capsule with the resulting mixture.

However, AVODART^{®} has a disadvantage in that it is inconvenient to take because the amount of excipients that make up the product is relatively large compared to the active ingredient, which increases the volume of the soft capsule.

Conventionally, studies have been conducted on ways to reduce the volume of an oral formulation containing dutasteride, thereby increasing dosing convenience and improving dissolution stability.

That is, in the conventional art, there have been attempts to increase dosing convenience by reducing the volume, but there is a problem in that dosing convenience is low because daily dosing is needed.

Accordingly, there is an urgent need to develop a therapeutic agent for prostatic hyperplasia, prostate cancer, and alopecia that overcomes the problems of the conventional formulation containing dutasteride, may maintain its efficacy for 3 months or more by a single administration, and is easy to store and handle.

### [Prior Art Documents]

### [Patent Documents]

(Patent Document 1) KR 10-1833280 B1

### DISCLOSURE

### Technical Problem

An object of the present invention is to provide a sustained-release injectable composition containing dutasteride.

Another object of the present invention is to provide a sustained-release injectable composition containing dutasteride, which may exhibit a sustained dutasteride release effect for 3 months or more even when the dose of dutasteride is equal to or less than that of existing AVODART^{®}, which is taken once a day, thereby exhibiting a long-acting drug effect by a single injection.

Still another object of the present invention is to provide an injectable composition containing sustained-release particles, which may exhibit a therapeutic effect on prostatic hyperplasia, prostate cancer, and hair loss continuously for 3 months or more, may maintain an effective concentration of the drug at a constant level by controlling the release of the drug due to the constant average diameter of the microparticles contained in the sustained-release injectable composition, and may reduce foreign body sensation and pain when administered by injection to a patient.

### Technical Solution

To achieve the above object, the present invention provides a sustained-release injectable composition containing dutasteride, which contains 8 mg to 100 mg of dutasteride, wherein the dutasteride is uniformly distributed in microparticles, wherein the microparticles release the dutasteride in a sustained manner for 3 to 6 months after being injected *in vivo,* and the average diameter of the microparticles is 30 µm to 90 µm.

The injectable composition may exhibit a maximum plasma concentration (Cₘₐₓ) of the dutasteride one week or more after being injected into a beagle dog.

The maximum plasma concentration (Cₘₐₓ) value of the dutasteride is 200 ng/mL to 1,100 ng/mL.

The microparticles may comprise two or more biodegradable polymers selected from the group consisting of polylactic acid, polylactide, polylactide-co-glycolide (PLGA), polyphosphazine, polyiminocarbonate, polyphosphoester, polyanhydride, polyorthoester, polycaprolactone, polyhydroxyvalerate, polyhydroxybutyrate, and polyamino acids.

The biodegradable polymers may comprise polylactic acid and polylactide-co-glycolide (PLGA) at a weight ratio of 1:1 to 1:4.

The microparticles may comprise the dutasteride and the biodegradable polymers at a weight ratio of 1:2 to 1:5.

The microparticles may release less than 50 wt% of the dutasteride for 24 hours, as a result of conducting an accelerated release test under the following conditions:

### [Test conditions]

Water containing 1% sodium lauryl sulfate is used as a dissolution test solution, the microparticles are mixed with the dissolution test solution, a shaking water bath is used as a dissolution tester, and a glass test container with a capacity of 120 ml is used as a dissolution test container, and shaking is performed at a speed of 120 rpm at 45°C.

The microparticles may further comprise a coating layer on the outer surface thereof.

The standard deviation (SD) for the diameter of the microparticles is 2 to 7.

The width of the peak in the results of particle size analysis (PSA) of the microparticles is 5 to 15.

### Advantageous Effects

In addition, the sustained-release injectable composition of the present invention may exhibit a therapeutic effect on prostatic hyperplasia, prostate cancer, and hair loss continuously for 3 months or more, may maintain an effective concentration of the drug at a constant level by controlling the release of the drug due to the constant average diameter of the microparticles contained in the sustained-release injectable composition, and may reduce foreign body sensation and pain when administered by injection to a patient.

### Brief Description of Drawings

FIG. 1 shows the results of an accelerated release test for microparticles according to one embodiment of the present invention.
FIG. 2 shows the results of pharmacokinetic (PK) analysis of a sustained-release injectable composition according to one embodiment of the present invention.
FIG. 3 shows the results of PK analysis of a sustained-release injectable composition according to one embodiment of the present invention.
FIG. 4 shows the results of PK analysis of a sustained-release injectable composition according to one embodiment of the present invention.
FIG. 5 is a SEM photograph of microparticles according to one embodiment of the present invention.
FIG. 6 is a SEM photograph of microparticles according to one embodiment of the present invention.
FIG. 7 is a SEM photograph of aggregated microparticles according to one embodiment of the present invention.

### Best Mode

The present invention relates to a sustained-release injectable composition containing dutasteride, which contains 8 mg to 100 mg of dutasteride, wherein the dutasteride is uniformly distributed in microparticles, wherein the microparticles release the dutasteride in a sustained manner for 3 to 6 months after being injected *in vivo,* and the average diameter of the microparticles is 30 µm to 90 µm.

### Mode for Invention

Hereinafter, embodiments of the present invention will be described in detail so that those skilled in the art can easily implement the present invention. However, the present invention may be embodied in various different forms and is not limited to the embodiments described herein.

The present invention relates to a sustained-release injectable composition containing dutasteride, characterized in that it may exhibit a sustained drug release effect for 3 months or more by a single injection.

As the sustained-release injectable composition exhibits a sustained drug release effect for 3 months or more with a single injection as described above, it may exhibit a therapeutic effect on benign prostatic hyperplasia, prostate cancer, and hair loss, and thus replace an existing product that is taken once a day, thereby significantly improving dosing convenience.

To achieve the above characteristic, specifically, the sustained-release injectable composition containing dutasteride according to the present invention contains 8 mg to 100 mg of dutasteride, wherein the dutasteride is uniformly distributed in microparticles, wherein the microparticles release the dutasteride in a sustained manner for 3 to 6 months after being injected *in vivo,* and the average diameter of the microparticles may be 30 to 90 µm.

The sustained-release injectable composition of the present invention contains dutasteride, and is characterized in that a single dosage form contains 8 mg to 100 mg of the dutasteride.

AVODART^{®}, which is taken once a day, contains 0.5 mg of dutasteride in a single dosage form. Based on 84 days of administration (3 months), a total of 42 mg is administered.

The present invention is characterized by allowing dutasteride to be released in a sustained manner for a long period of time even when dutasteride is contained in a dose equivalent to or lower than that of AVODART^{®}.

Accordingly, the sustained-release injectable composition of the present invention is characterized by releasing dutasteride in a sustained manner for 3 to 6 months after being injected *in vivo.* For example, in the case of a 3-month sustained-release formulation, the sustained-release injectable composition may contain 8 to 42 mg, 15 to 42 mg, or 25 to 42 mg of dutasteride. Even when the composition contains dutasteride within the above range, it may exhibit the effect of releasing dutasteride in a sustained manner for 3 months.

In addition, in the case of a 6-month sustained-release formulation, the composition may contain 16 mg to 84 mg, 30 mg to 84 mg, or 50 mg to 84 mg of dutasteride. Even when the composition contains dutasteride within the above range, it may exhibit the effect of releasing dutasteride in a sustained manner for 3 months.

The sustained-release injectable composition contains microparticles containing dutasteride, wherein the microparticles are uniform spherical particles and contain dutasteride uniformly.

As described below, the microparticles of the present invention are spherical particles having a uniform diameter, and pluralities of such particles are contained in the sustained-release injectable composition. As the composition contains the particles having a uniform diameter as described above, the composition may exhibit the effect of releasing dutasteride in a sustained manner after being injected *in vivo,* and may control the release timing of dutasteride, etc.

That is, if the size of the particles is not uniform, a problem may arise in that the *in vivo* degradation rates of the particles may be different from each other, and thus the release rate of dutasteride contained in the particles may not be controlled. Accordingly, the present invention is characterized by containing only particles having a uniform diameter, so that it may control the release rate of dutasteride, and thus exhibit the effect of releasing dutasteride in a sustained manner for 3 to 6 months.

The average diameter of the microparticles may be 30 µm to 90 µm, 35 µm to 85 µm, or 40 µm to 80 µm. By containing uniform particles having an average diameter within the above range, the composition may prevent the over-release of dutasteride in an initial stage after being injected *in vivo,* and may release dutasteride after a certain period of time so that dutasteride reaches a maximum plasma concentration, thereby exhibiting the effect of releasing dutasteride in a sustained manner for 3 to 6 months.

Specifically, the injectable composition may exhibit a maximum plasma concentration (Cₘₐₓ) of dutasteride 1 week or more after being injected into a beagle dog, and specifically, may exhibit a maximum plasma concentration (Cₘₐₓ) within 2 to 5 weeks after injection.

As described above, the sustained-release injectable composition of the present invention is characterized in that, after being injected into a beagle dog, it releases dutasteride in a sustained manner, exhibits a maximum plasma concentration (Cₘₐₓ) value of dutasteride one week or more after injection, and then releases dutasteride for a desired period of time with a steady decrease in the blood concentration of dutasteride.

That is, the sustained-release injectable composition according to the present invention is characterized in that, after being injected into a beagle dog, it exhibits a maximum plasma concentration (Cₘₐₓ) value of dutasteride after 1 week or within 2 weeks to 5 weeks after injection so that it exhibits the effect of releasing dutasteride in a sustained manner for 3 to 6 months.

According to the results of release of dutasteride as described above, after the injectable composition of the present invention is injected into a beagle dog, the release amount of dutasteride is small within 1 day, that is, within 24 hours, but the release amount of dutasteride increases continuously thereafter, reaches a maximum value after 1 week or within 2 to 5 weeks, and then continuously decreases, so that the release of dutasteride is terminated at about 3, 4, 5, or 6 months.

More specifically, as a result of measuring the plasma concentration of dutasteride after injecting the sustained-release injectable composition of the present invention into a beagle dog, the maximum plasma concentration (C₂₄ₕ) of dutasteride at 24 hours may be 4 ng/mL to 40 ng/mL, 5 ng/mL to 38 ng/mL, 6 ng/mL to 35 ng/mL, or 8 ng/mL to 32 ng/mL.

In addition, the maximum plasma concentration (Cₘₐₓ) of dutasteride 1 week or more after injection may be 200 ng/mL to 2,500 ng/mL, 400 ng/mL to 2,500 ng/mL, or 500 ng/mL to 2,500 ng/mL. The maximum plasma concentration (Cₘₐₓ) of dutasteride may differ depending on whether the sustained-release injectable composition of the present invention is a 3-month sustained-release formulation, a 4-month sustained-release formulation, a 5-month sustained-release formulation, or a 6-month sustained-release formulation. Specifically, in the case of a 3-month dosage form, the maximum plasma concentration (Cₘₐₓ) of dutasteride may be 500 ng/mL to 1,100 ng/mL. That is, in order to provide a dosage form that releases dutasteride in a sustained manner for a longer period of time, the content of dutasteride in the injectable composition is increased, and for this reason, when the injectable composition is administered to a beagle dog, the maximum plasma concentration (Cₘₐₓ) of dutasteride may also change.

As a result of analyzing the plasma concentration of dutasteride after injecting the sustained-release injectable composition of the present invention into a beagle dog, it can be clearly confirmed that the amount of dutasteride released is small within 24 hours, and the maximum plasma concentration (Cₘₐₓ) is reached one week or more after injection. When the composition exhibits the blood concentration value of dutasteride as above, it may exhibit the effect of releasing dutasteride in a sustained manner for 3 months or more, or 3 to 6 months.

The microparticles may comprise two or more biodegradable polymers selected from the group consisting of polylactic acid, polylactide, polylactide-co-glycolide (PLGA), polyphosphazine, polyiminocarbonate, polyphosphoester, polyanhydride, polyorthoester, polycaprolactone, polyhydroxyvalerate, polyhydroxybutyrate, and polyamino acids. Specifically, the microparticles may comprise polylactic acid and polylactide-co-glycolide as biodegradable polymers.

When the microparticles are produced to comprise two or more biodegradable polymers as described above, the biodegradable polymers that biodegrade over different periods of time are used, and thus may exhibit the effect of releasing dutasteride by being degraded over a desired period of time.

That is, when only one biodegradable polymer that biodegrades over a short period of time is used, it may exhibit the effect of releasing dutasteride only for a short period of time.

In addition, when a biodegradable polymer that biodegrades over a long period of time is used, it may exhibit the effect of releasing dutasteride for a longer period of time, but there is a problem in that, in an initial stage after administration by injection, the amount of release of dutasteride is excessively small, making it difficult for dutasteride to exhibit efficacy. That is, in order to exhibit the efficacy of dutasteride *in vivo,* the plasma concentration value of dutasteride should be a certain level or higher. However, if a biodegradable polymer that biodegrades over a long period of time is used, there is a problem in that, in an initial stage after administration by injection, the degree of degradation of dutasteride is insufficient, making it difficult for dutasteride to exhibit efficacy.

The biodegradable polymers may comprise polylactic acid and polylactide-co-glycolide (PLGA) at a weight ratio of 1:1 to 1:4, or 1:2 to 1:3. When the biodegradable polymers are used in combination at the above-described weight ratio, they may exhibit the effect of releasing dutasteride for a desired period of time as described above.

The microparticles may comprise dutasteride and the biodegradable polymers at a weight ratio of 1:2 to 1:5, 1:2 to 1:4, or 1:2 to 1:3. When dutasteride and the biodegradable polymers are used in combination at the above weight ratio, dutasteride may be released in a sustained manner over a long period of time by degradation of the biodegradable polymers.

The microparticles may release less than 50 wt% of dutasteride for 24 hours, as a result of conducting an accelerated release test under the following conditions:

### [Test conditions]

Water containing 1% sodium lauryl sulfate is used as a dissolution test solution, the microparticles are mixed with the dissolution test solution, a shaking water bath is used as a dissolution tester, and a glass test container with a capacity of 120 ml is used as a dissolution test container, and shaking is performed at a speed of 120 rpm at 45°C.

The accelerated release test is a test conducted to examine the extent of sustained drug release after producing particles containing the drug using the biodegradable polymers. In other words, it is a test conducted to indirectly examine whether the drug may be released in a sustained manner for a long period of time.

The present invention is characterized in that the microparticles release less than 50 wt% of dutasteride for 20 hours, as a result of conducting the accelerated release test under the above-described conditions.

As described above, the microparticles of the present invention may exhibit the effect of releasing dutasteride in a sustained manner for 3 to 6 months by maintaining the release level of dutasteride at less than 50 wt% for 20 hours, as a result of conducting the accelerated release test.

Why the release level of dutasteride may be controlled as described above may be that two or more biodegradable polymers are contained in the microparticles. As described below, it can be seen that, when only one biodegradable polymer is used, it exhibits the effect of releasing 70 wt% or more of dutasteride for 20 hours. That is, when if microparticles containing dutasteride are produced using only one biodegradable polymer, a problem may arise in that the microparticles may not exhibit the effect of dutasteride in a sustained manner for 3 to 6 months as in the present invention.

In contrast, the microparticles of the present invention may exhibit the effect of releasing dutasteride in a sustained manner for a long period of time, since the point in time at which 80 wt% of dutasteride is released is 50 hours or more or 70 hours or more.

The microparticles may further comprise a coating layer on the outer surface thereof. The coating layer may be formed by coating with mannitol. Where the surface of the microparticles is coated with mannitol to form a coating layer as described above, the coating layer may improve the flowability of the microparticles when filling for use as an injectable formulation, so that only a desired amount of the microparticles may be efficiently filled. In addition, when the microparticles having the coating layer formed using mannitol as described above do not aggregate together when freeze-dried.

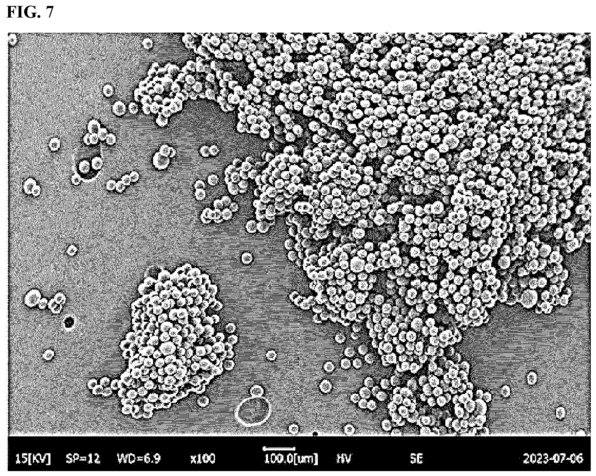

In contrast, microparticles that do not have the coating layer formed thereon, when freeze-dried, may aggregate together as shown in FIG. 7. In addition, the flowability during filling is poor, and thus differences in the degree of inclusion of microparticles may appear.

As described above, the microparticles of the present invention may have an average diameter of 30 µm to 90 µm, 35 µm to 85 µm, or 40 µm to 80 µm. In addition, the standard deviation (SD) for the average diameter of the microparticles may be 2 to 7. The standard deviation (SD) refers to the degree of distribution of the average diameter, and it can be said that a smaller standard deviation (SD) means a more uniform particle size.

In addition, the width of the peak in the results of PSA may be 5 to 15. The width of the peak refers to the width of each peak (=width of 16 to 84%), and as described below, the microparticles produced by the production method of the present invention do not show a plurality of peaks in PSA. This means that the produced microparticles themselves are very uniform in size.

By comprising uniform particles having a peak width within the above range, the over-release of dutasteride in an initial stage after being injected *in vivo* may be prevented, and the particles may exhibit a maximum plasma concentration of dutasteride by releasing dutasteride after a certain period of time, thereby exhibiting the effect of releasing dutasteride in a sustained manner for 3 to 6 months.

A method for preparing a sustained-release injectable composition containing dutasteride according to another embodiment of the present invention may comprise: step 1) of preparing an oil phase solution by mixing dutasteride and biodegradable polymers; step 2) of preparing an aqueous phase solution by dissolving a surfactant in a solvent; step 3) introducing the oil phase solution and the aqueous phase solution into a first microchannel and a second microchannel, respectively, which have an intersection formed therebetween, and allowing the oil phase solution and the aqueous phase solution to flow, thereby producing microparticles at the intersection; step 4) of collecting the microparticles in a bath containing the aqueous phase solution; step 5) of removing an organic solvent from the collected microparticles; and step 6) of washing the microparticles, from which the organic solvent has been removed, with purified water, followed by drying; and step 7) of mixing the dried microparticles with a suspending solvent.

Step 1) is a step of preparing an oil phase solution by dissolving dutasteride and biodegradable polymers in an organic solvent. Here, the biodegradable polymers may be two or more biodegradable polymers selected from the group consisting of polylactic acid, polylactide, polylactide-co-glycolide (PLGA), polyphosphazine, polyiminocarbonate, polyphosphoester, polyanhydride, polyorthoester, polycaprolactone, polyhydroxyvalerate, polyhydroxybutyrate, and polyamino acids, and are preferably polylactide-co-glycolide (PLGA) and polylactide (PLA), but the biodegradable polymers are not limited to the above examples.

In addition, the organic solvent is water-immiscible, and may be, for example, any one or more selected from the group consisting of chloroform, chloroethane, dichloroethane, trichloroethane, and mixtures thereof, with dichloromethane being preferred, but the organic solvent is not limited to the above examples. In addition to the above-listed organic solvents, any organic solvent may be used without limitation, as long as it is capable of dissolving the biodegradable polymers and dutasteride and may be easily selected by those skilled in the art.

Step 1) is a step of preparing the oil phase solution by dissolving dutasteride and the biodegradable polymers and as the solvent, the organic solvent described above is used. The organic solvent is used to completely dissolve dutasteride and the biodegradable polymers based on the dissolution properties thereof.

The weight ratio between dutasteride and the biodegradable polymers in the oil phase solution may be 1:2 to 1:5, 1:2 to 1:4, or 1:2 to 1:3. When dutasteride and the biodegradable polymers are used in combination at a weight ratio within the above range, dutasteride may be released in a sustained manner for a long period of time by degradation of the biodegradable polymers.

If the weight ratio between dutasteride and the biodegradable polymers is less than 1:1, that is, if the content of the biodegradable polymers is lower than the above weight ratio, a problem may arise in that, because the content of the biodegradable polymers is lower than the content of dutasteride, it is difficult to produce sustained-release microparticles in which dutasteride is uniformly distributed in spherical biodegradable polymer particles. If the weight ratio between the biodegradable polymers and dutasteride is more than 1:5, that is, if the content of the biodegradable polymers is more than the above weight ratio, a problem may arise in that, because the content of dutasteride in the sustained-release microparticles is low, a large amount of the sustained-release microparticles need to be administered in order to administer the drug at a desired concentration.

More specifically, the content of the biodegradable polymers in the oil phase solution is 15 to 25 wt%, preferably 20 wt%, without being limited thereto.

Step 2) is a step of preparing an aqueous phase solution by dissolving a surfactant in water. As the surfactant, any surfactant may be used without limitation as long as it may help the oil phase solution containing the biodegradable polymers and dutasteride to form a stable emulsion. Specifically, the surfactant may be any one or more selected from the group consisting of nonionic surfactants, anionic surfactants, cationic surfactants, and mixtures thereof, and more specifically, may be any one or more selected from the group consisting of methylcellulose, polyvinylpyrrolidone, lecithin, gelatin, polyvinyl alcohol, polyoxyethylene sorbitan fatty acid ester, polyoxyethylene castor oil derivatives, sodium lauryl sulfate, sodium stearate, ester amine, linear diamine, fatty amines, and mixtures thereof, with polyvinyl alcohol being preferred, but the surfactant is not limited to the above examples.

The aqueous phase solution may contain the surfactant in an amount of 0.1 to 1.0 wt%, 0.1 to 0.5 wt%, or 0.25 wt%, with the remainder being water.

Step 3) is a step of introducing the oil phase solution and the aqueous phase solution into microchannels formed on a wafer and allowing the oil phase solution and the aqueous phase solution to flow therethrough.

More specifically, the microchannels may be formed on a material selected from the group consisting of a silicon wafer and a polymer film, but the material is not limited to the above examples, and it is possible to use any material on which the microchannels may be formed.

The polymer film may be selected from the group consisting of polyimide, polyethylene, fluorinated ethylene propylene, polypropylene, polyethylene terephthalate, polyethylene naphthalate, polysulfone, and mixtures thereof, without being limited thereto.

As an example, aluminum is deposited on a silicon wafer using an e-beam evaporator, and photoresist is patterned on the aluminum using a photolithography technique. Thereafter, the aluminum is etched using the photoresist as a mask, the photoresist is removed, the silicon wafer is etched by deep ion reactive etching (DRIE) using the aluminum as a mask, the aluminum is removed, and then glass is anodically bonded onto the wafer and hermetically sealed, thereby fabricating the microchannels.

The average diameter of the microchannels varies depending on whether the chip is a 7-channel chip or a 140-channel chip. As microchannels for fabricating the microparticles of the present invention, a 140-channel chip was used. In the case of the 140-channel chip, the average diameter of the channels when the oil phase solution and the aqueous phase solution are introduced into the microchannels is 300 µm to 500 µm, and the oil phase solution and the aqueous phase solution pass through the respective channels and then pass through resistance channels. The average diameter of the resistance channels is 10 µm to 50 µm. After passing through the resistance channels, the oil phase solution and the aqueous phase solution pass through a junction channel where they intersect, and the diameter of the junction channel may be 50 µm to 150 µm. After the oil phase solution and the water solution intersect each other within the junction channel to form an emulsion, they immediately pass through a microchannel with a diameter of 70 µm to 90 µm, and then pass through a microchannel with a diameter of 200 µm to 300 µm. If the average diameter of the junction channels is 50 µm or less, the produced sustained-release particles may have a small diameter of less than 30 µm, which may affect the release and *in vivo* uptake of the effective drug. In addition, if the average size of the produced sustained-release particles exceeds 100 µm, foreign body sensation and pain may increase when administered by injection, and the particle size distribution of the produced particles becomes large, making it difficult to produce sustained-release particles with a uniform particle size.

However, the average diameter of the microchannels may change depending on the range of the introduction pressure. In addition, the average diameter of the microchannels is closely related not only to the average diameter of the particles, but is also to the introduction pressure of the oil phase solution and the aqueous phase solution.

Step 3) is a step of introducing the oil phase solution and the aqueous phase solution into the first microchannel and the second microchannel, respectively, which have an intersection formed therebetween, and allowing the oil phase solution and the aqueous phase solution to flow under pressure conditions.

That is, the oil phase solution flows along the first microchannel, and the aqueous phase solution flows along the second microchannel configured to form an intersection with the first microchannel, and meets the flow of the oil phase solution.

More specifically, the oil phase solution may be introduced into the first microchannel under a pressure of 200 to 800 mbar, and the pressure may be increased at a first rate of 1 to 5 mbar/min, and when the pressure reaches 500 to 1,000 mbar, it may be increased at a second rate of 1 to 5 mbar/min.

In addition, the aqueous phase solution may be introduced into the second microchannel under a pressure which is 4 to 12 times higher than the pressure under which the oil phase solution is introduced into the first microchannel.

Specifically, in the production method using the microchannels, when the flow rates of the oil phase solution and the aqueous phase solution flowing inside the microchannels were set to certain values using a flow meter and the pressure was measured through feedback control, it was confirmed that the pressure required for the oil phase solution to flow through the microchannel at a certain flow rate increased gradually over time.

Therefore, it is possible to minimize the flow rate variability by using a method of constantly increasing the pressure applied to the oil phase solution, and to prevent the problem of non-uniform microparticle distribution or channel closure due to slow curing of the oil phase solution inside the microchannel, and to increase the production yield of desired microparticles.

In addition, the pressure conditions used when introducing the oil phase solution and the aqueous phase solution into the microchannels serve to control the average diameter of the produced microparticles, and if the above-described range is not specifically satisfied, a problem may arise in that the size of the produced particles is not uniform, or the average diameter range of the microparticles of the present invention is not satisfied, or the value of Equation 1 above is not satisfied.

That is, in order to increase the flow rate of the aqueous phase solution, which forms an intersection with the flow of the oil phase solution, compared to the flow rate of the oil phase solution introduced into the microchannel, the aqueous phase solution is allowed to flow under a higher pressure.

As described above, when the flow rates of the oil phase solution and the aqueous phase solution are made different from each other and the flow rate of the aqueous phase solution is increased compared to the flow rate of the oil phase solution, the aqueous phase solution having a relatively higher flow rate compresses the oil phase solution at the point where the flow of the oil phase solution and the flow of the aqueous phase solution meet each other, and in this case, due to repulsive force between the oil phase solution and the aqueous phase solution, the biodegradable polymers and dutasteride in the oil phase solution form spherical microparticles, and more specifically, form microparticles in which dutasteride is uniformly distributed in the spherical biodegradable polymers.

Step 4) is a step of collecting microparticles. In this step, the microparticles are collected in a bath containing the aqueous phase solution to prevent aggregation of initially produced microparticles.

Step 4) is performed using the aqueous phase solution prepared in step 2), that is, a mixed solution of the surfactant and water. Specifically, a portion of the aqueous phase solution prepared in step 2) is introduced into the microchannel, and the other portion is transferred into the bath in step 4) and used to prevent aggregation of the collected microparticles.

Step 5) is a step of removing an organic solvent from the microparticles collected in the bath. In this step, an organic solvent present on the surfaces of the sustained-release microparticles is evaporated and removed by stirring the microparticles at a predetermined stirring speed at a predetermined temperature. Specifically, step 5) may include steps of: 5-1) subjecting the microparticles to first stirring at a speed of 100 to 300 rpm at 15 to 20°C for 20 to 40 minutes; 5-2) subjecting the microparticles to second stirring at a speed of 100 to 300 rpm at 30 to 40°C for 60 to 120 minutes; and 5-3) subjecting the microparticles to third stirring at a speed of 100 to 300 rpm at 40 to 45°C for 4 to 8 hours

The first and second stirring steps are performed at different stirring speeds at different temperatures for different stirring times.

As described above, step 5) is characterized in that the stirring temperature is higher in the second stirring step than in the first stirring step. As the stirring temperature is increased stepwise, it is possible to control the evaporation rate of the organic solvent present on the surfaces of the microparticles. That is, it is possible to slowly evaporate the organic solvent present on the surfaces of the microparticles, thereby producing microparticles.

The temperature at which the oil phase solution and the aqueous phase solution flow through the microchannels is also 15 to 20°C, preferably 17°C. That is, after the oil phase solution and the aqueous phase solution flow through the microchannels and microparticles are produced at an intersection therebetween, the collected microparticles are constantly maintained at a low temperature of 15 to 20°C until they are stirred in the first stirring step. It is possible to produce and maintain spherical particles only when microparticles are maintained at low temperature during the production thereof. That is, if a low-temperature condition is not used, a problem arises in that it is difficult to produce particles having a uniform spherical shape.

Thereafter, in the second stirring step and the third stirring step, the temperature is increased gradually and the stirring time is increased so that the organic solvent present on the surfaces of the microparticles may be slowly evaporated, thereby minimizing the effect of the organic solvent on the surfaces of the microparticles by evaporation of the organic solvent from the surface. That is, if the organic solvent is rapidly evaporated, a problem may arise in that the surfaces of the microparticles are not smooth and become rough due to evaporation of the organic solvent. In order to prevent this problem, the evaporation rate of the organic solvent may be controlled by increasing the temperature gradually as described above and also increasing the stirring process time, and due to this control of the evaporation rate of the organic solvent, it is possible to control the surface roughness of the produced microparticles.

Lastly, step 6) is a step of washing and drying the microparticles. In this step, the microparticles from which the organic solvent on the surface has been completely removed by stirring are washed several times with sterile filtered purified water to remove the surfactant remaining on the microparticles.

On the microparticles from which the remaining surfactant has been removed, a coating layer may be formed using an aqueous mannitol solution. Specifically, a mannitol aqueous solution may be added to the microparticles from which the surfactant has been removed, thereby forming a mannitol coating layer on the outer surface of the microparticles, followed by freeze-drying.

The finally produced microparticles are in a form in which dutasteride is uniformly distributed in microparticles made of spherical biodegradable polymers, and may contain dutasteride and the biodegradable polymers at a weight ratio of 1:2 to 1:5.

The weight ratio between dutasteride and biodegradable polymers contained in the microparticles is the same as the weight ratio in the oil phase solution. Specifically, as the organic solvent is completely evaporated and removed from the produced microparticles, the produced microparticles may contain dutasteride and the biodegradable polymers at the same weight ratio as the weight ratio in the oil phase solution.

The produced microparticles may be prepared into an injectable composition by mixing with a suspending solvent.

The suspending solvent contains an isotonic agent, a suspending agent, and a solvent.

More specifically, the isotonic agent may be selected from the group consisting of D-mannitol, maltitol, sorbitol, lactitol, xylitol, sodium chloride, and mixtures thereof, with D-mannitol being preferred, but the isotonic agent is not limited to the above examples.

The suspending agent is selected from the group consisting of sodium carboxymethylcellulose, polysorbate 80, starch, starch derivatives, polyhydric alcohols, chitosan, chitosan derivatives, cellulose, cellulose derivatives, collagen, gelatin, hyaluronic acid (HA), alginic acid, algin, pectin, carrageenan, chondroitin, chondroitin sulfate, dextran, dextran sulfate, polylysine, titin, fibrin, agarose, fluran, xanthan gum, and mixtures thereof, with sodium carboxymethylcellulose and polysorbate 80 being preferred, but the suspending agent is not limited to the above examples.

As the solvent, water for injection may be used, and any solvent that may be used as water for injection may be used without limitation.

### Production Example 1

### Production of Microparticles Containing Dutasteride

An oil phase solution was prepared by dissolving dutasteride, PLGA, and PLA in dichloromethane. The biodegradable polymers in the oil phase solution may include PLGA and PLA at a weight ratio of 2:1, and dutasteride and the biodegradable polymers may be included at a weight ratio of 1:3.

An aqueous phase solution containing 0.25 wt% of polyvinyl alcohol was prepared by mixing polyvinyl alcohol as a surfactant with water.

The oil phase solution and the aqueous phase solution were introduced into microchannels formed on a silicon wafer and allowed to flow.

Here, in order to allow each of the oil phase solution and the aqueous phase solution to flow at a constant flow rate, the oil phase solution was allowed to flow under a pressure of 650 mbar, which was increased at a constant rate of 2 mbar/min, and the aqueous phase solution was allowed to flow under a pressure of 2,800 mbar. The temperature was maintained at 17°C, and the stirring speed was maintained at 300 rpm.

Microparticles produced at the intersection between the flow of the oil phase solution and the flow of the aqueous phase solution were collected in a bath containing the aqueous phase solution. The microparticles collected in the bath were subjected to first stirring at a speed of 300 rpm for 30 min at 17°C, and then subjected to second stirring at a speed of 400 rpm for 1 hour at an increased temperature of 35°C, and then subjected to third stirring at a speed of 500 rpm for 5 hours at an increased temperature of 43°C

After completion of stirring, the microparticles were washed several times with sterile filtered purified water and freeze-dried, thereby producing microparticles having an average diameter (D₅₀) of 40 µm.

### Production Example 2

Microparticles were produced in the same manner as in Production Example 1, except that the biodegradable polymers in the oil phase solution included PLGA and PLA at a weight ratio of 1:1, and dutasteride and the biodegradable polymers were included at a weight ratio of 1:2.

### Production Example 3

Microparticles were produced in the same method as in Production Example 1, except that only PLGA was used as a biodegradable polymer.

### Production Example 4

Microparticles were produced in the same method as in Production Example 3, except that the average diameter of the microparticles was 80 µm.

### Production Example 5

Microparticles were produced in the same method as in Production Example 1, except that the average diameter of the microparticles was 80 µm.

### Production Example 6

Microparticles were produced in the same method as in Production Example 2, except that the average diameter of the microparticles was 80 µm.

### Experimental Example 1

### Accelerated Release Test

Water containing 1% sodium lauryl sulfate was used as a dissolution test solution, and the microparticles were mixed with the dissolution test solution. A shaking water bath was used as a dissolution tester, and a glass test container with a capacity of 120 ml was used as a dissolution test container. Shaking was performed at a speed of 120 rpm at 45°C.

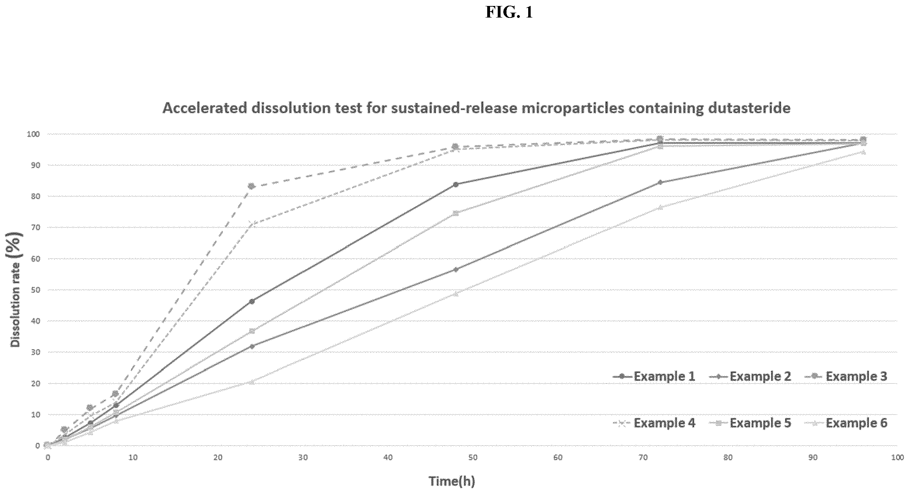

FIG. 1 shows the results of conducting the accelerated release test for the microparticles of Production Examples 1 to 6 as Examples 1 to 6.

Referring to the results of the test, it was confirmed that, when only one biodegradable polymer, PLGA, was used, the release level of dutasteride exceeded 50% at 24 hours. In light of the above result, the microparticles of Examples 3 and 4, which contain only PLGA, are used, there is a problem that it is difficult for the microparticles to exhibit the effect of releasing dutasteride in a sustained manner for 3 to 6 months.

On the other hand, as a result of conducting the accelerated release test for Examples 1, 2, 5 and 6, it can be confirmed that the time point at which 90% or more of dutasteride was released was 72 hours or more or close to 100 hours.

In addition, it can be confirmed that, in the case of Examples 1 and 5 and Examples 2 and 6, which were different only in the average particle diameter, the release rate of dutasteride was delayed as the particle diameter increased.

### Experimental Example 2

### Evaluation of Pharmacokinetic (PK) Characteristics

The microparticles of Production Example 1 were added to 1.5 ml (based on one vial) of a suspending solvent, and then uniformly suspended, thereby preparing a composition for subcutaneous injection.

The suspending solvent had the composition shown in Table 1 below.

**[Table 1]**

| Volume | Purpose of mixing | Component name | Quantity | Unit |
|---|---|---|---|---|
| 1.5 mL | Isotonic agent | D-Mannitol | 75.0 | mg |
| | Suspending agent | Sodium carboxymethylcellulose | 3.75 | mg |
| | Suspending agent | Polysorbate 80 | 1.5 | mg |
| | Solvent | Water for injection water | Remainder | |

Using the microparticles of Production Example 1, compositions for subcutaneous injection containing dutasteride in amounts of 8.4 mg (G2), 25.2 mg (G3), and 42 mg (G4), respectively, were prepared.

The microparticles of Production Example 2 were added to the above-described suspending solvent, thereby preparing a composition for subcutaneous injection (G5).

Avodart (GSK) soft capsule 0.5 mg was used as a Comparative Example (G1).

Each of G1 to G5 was administered to beagle dogs, and blood samples were collected to measure the plasma concentration (PK) of dutasteride. For the experiment, each of injectable compositions G2 to G5 was administered to five beagle dogs, and the administration route was subcutaneous injection. G1 was administered once to five beagles at the same time every day.

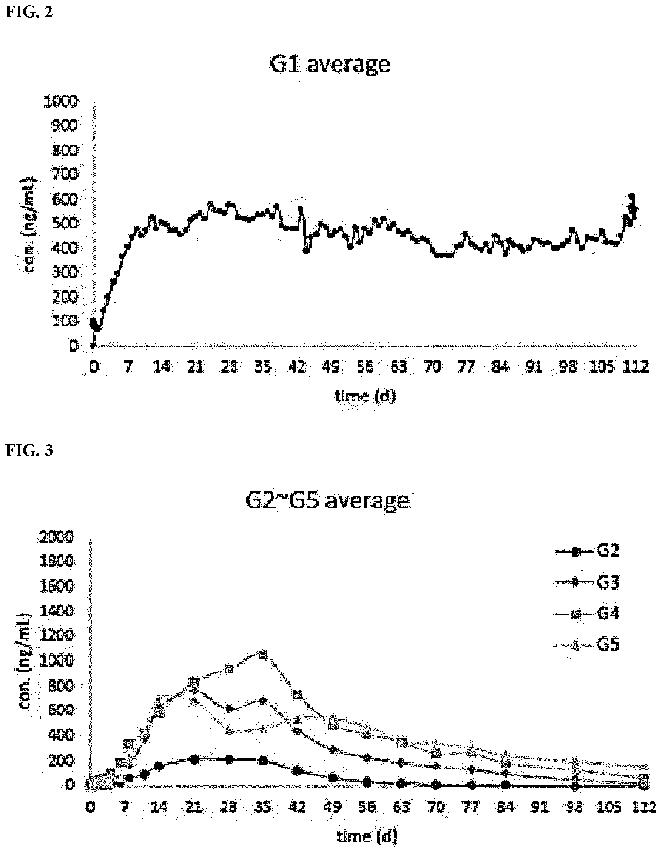

After blood collection, the average value of PKs for the five beagle dogs was calculated. The experimental results are shown in FIGS. 2 to 4.

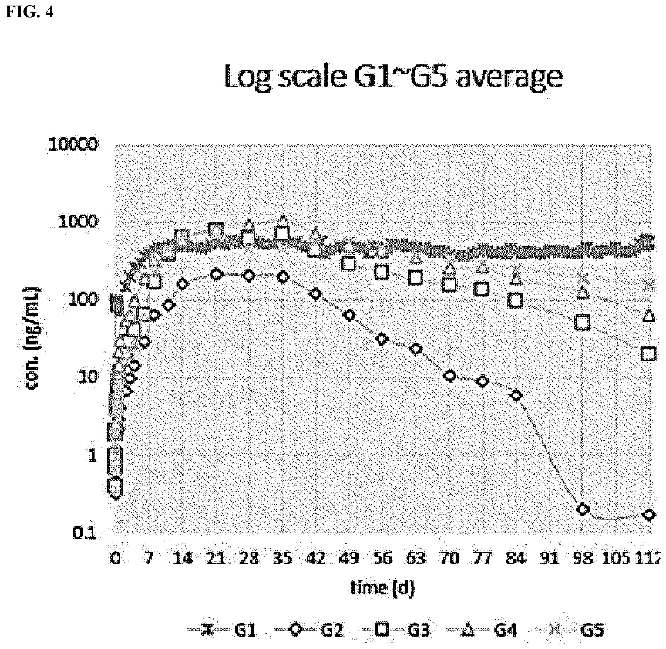

FIG. 2 shows the average PK measurement results for G1, and FIG. 3 shows the average PK measurement results for G2 to G5. FIG. 4 shows the analysis results on a log scale for G1 to G5.

It can be confirmed that G1 shows a constant PK value after 7 days as it is taken daily.

It can be confirmed that, in the case of G3 to G5, the plasma concentration of dutasteride continuously increased, reached the maximum plasma concentration after 1 week, and then decreased. However, it can be confirmed that, in the case of G2, the amount of dutasteride administered was excessively small, and the release of releasing dutasteride did not appear for 3 months.

The PK measurement results for G2 to G5 are as shown in Table 2 below:

**[Table 2]**

| Time (w) | Time (d) | Time (h) | G2 | G3 | G4 | G5 |
|---|---|---|---|---|---|---|
| 0.0 | 0.0 | 0 | No Peak | No Peak | No Peak | No Peak |
| 0.0 | 0.0 | 0.5 | 0.32325 | 0.3898 | 1.9858 | 0.36525 |
| 0.0 | 0.0 | 1 | 0.4186 | 0.9806 | 2.4434 | 0.5878 |
| 0.0 | 0.1 | 1.5 | 0.4966 | 0.685 | 2.6084 | 0.6906 |
| 0.0 | 0.1 | 2 | 0.588 | 0.8786 | 3.916 | 0.943 |
| 0.0 | 0.1 | 3 | 1.0458 | 1.875 | 5.2272 | 1.446 |
| 0.0 | 0.2 | 4 | 1.2242 | 2.1 | 6.1504 | 1.9622 |
| 0.0 | 0.3 | 6 | 2.1212 | 4.7562 | 9.9178 | 3.0732 |
| 0.0 | 0.3 | 8 | 2.0642 | 4.1252 | 11.5584 | 3.7702 |
| 0.1 | 0.4 | 10 | 2.0522 | 4.591 | 13.339 | 4.3352 |
| 0.1 | 0.5 | 12 | 2.1884 | 6.985 | 14.305 | 4.4168 |
| 0.1 | 0.8 | 18 | 2.9676 | 8.0476 | 21.5142 | 6.4272 |
| 0.1 | 1 | 24 | 4.0082 | 11.6678 | 30.1276 | 8.0218 |
| 0.3 | 2 | 48 | 6.7666 | 21.1678 | 53.6748 | 16.6622 |
| 0.4 | 3 | 72 | 9.8386 | 29.5632 | 64.0236 | 24.078 |
| 0.6 | 4 | 96 | 14.3968 | 40.6086 | 97.0532 | 27.0472 |
| 0.9 | 6 | 144 | 28.7132 | 64.3884 | 189.0906 | 64.633 |
| 1.1 | 8 | 192 | 64.3268 | 166.5396 | 333.4002 | 228.6784 |
| 1.6 | 11 | 264 | 85.1528 | 383.203 | 428.6358 | 438.0872 |
| 2 | 14 | 336 | 159.9186 | 637.5282 | 589.4112 | **700.6906** |
| 3 | 21 | 504 | **213.7886** | **770.474** | 836.1794 | 688.7982 |
| 4 | 28 | 672 | 209.102 | 617.7678 | 936.663 | 447.7378 |
| 5 | 35 | 840 | 202.3858 | 686.4664 | **1050.591** | 463.2668 |
| 6 | 42 | 1008 | 119.6382 | 446.0882 | 733.6584 | 542.6508 |
| 7 | 49 | 1176 | 64.9644 | 293.663 | 490.3916 | 541.1552 |
| 8 | 56 | 1344 | 31.7436 | 228.8524 | 414.9682 | 473.937 |
| 9 | 63 | 1512 | 23.5696 | 188.089 | 354.3624 | 351.9552 |
| 10 | 70 | 1680 | 10.7034 | 154.5424 | 259.0024 | 340.6144 |
| 11 | 77 | 1848 | 9.1374 | 134.1624 | 265.6142 | 303.364 |
| 12 | 84 | 2016 | 5.96525 | 95.9516 | 186.5662 | 246.6816 |
| 14 | 98 | 2352 | 0.199667 | 50.8484 | 127.0994 | 191.2418 |
| 16 | 112 | 2688 | 0.171 | 20.0644 | 63.4776 | 157.2748 |

According to the above measurement results, it can be confirmed that the maximum plasma concentration of dutasteride was reached between 2 and 5 weeks.

### Experimental Example 3

### Examination of Appearance of Microparticles

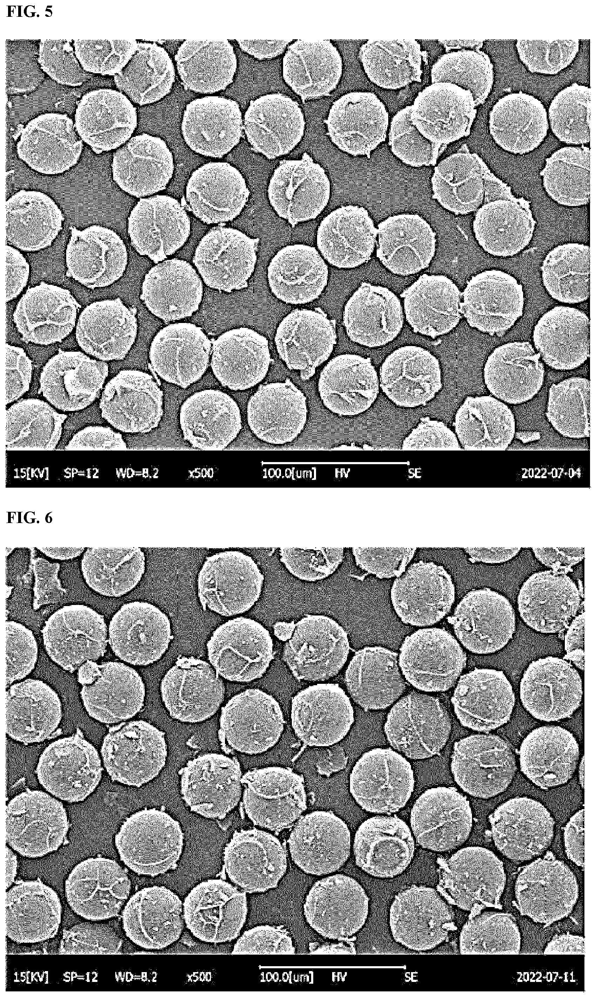

In Production Examples 1 and 2, mannitol coating was performed, and it was checked whether the mannitol coating layer was formed. Specifically, after the surfactant was removed from the microparticles of Production Examples 1 and 2, an aqueous mannitol solution was added to form a mannitol coating layer on the outer surface of the microparticles, followed by freeze-drying. It can be confirmed that the microparticles of Production Examples 1 and 2 had a mannitol coating layer formed thereon as shown in FIGS. 5 and 6.

On the other hand, the microparticles of Production Example 1 that were not coated with mannitol aggregated together as shown in FIG. 7.

The results of particle size analysis (PSA) of the microparticles produced in Production Examples 1 to 6 are as shown in Table 3 below:

**[Table 3]**

| | D50 | SD | Width |
|---|---|---|---|
| Production Example 1 | 40.66 | 2.648 | 5.3 |
| Production Example 2 | 40.91 | 2.714 | 5.43 |
| Production Example 3 | 40.71 | 2.785 | 5.57 |
| Production Example 4 | 81.06 | 5.29 | 10.57 |
| Production Example 5 | 80.64 | 5.29 | 10.57 |
| Production Example 6 | 82.77 | 6.58 | 13.16 |

Although the preferred embodiments of the present invention have been described in detail above, the scope of the present invention is not limited thereto, and various modifications and improvements made by those skilled in the art using the basic concept of the present invention as defined in the appended claims also fall within the scope of the present invention.

### Industrial Applicability

The present invention relates to a sustained-release injectable composition containing dutasteride.

## Claims

1. A sustained-release injectable composition containing dutasteride, which contains 8 mg to 100 mg of dutasteride, wherein the dutasteride is uniformly distributed in microparticles, wherein the microparticles release the dutasteride in a sustained manner for 3 to 6 months after being injected *in vivo,* and the microparticles have an average diameter of 30 µm to 90 µm.

2. The sustained-release injectable composition containing dutasteride according to claim 1, wherein the injectable composition exhibits a maximum plasma concentration (Cₘₐₓ) of the dutasteride one week or more after being injected into a beagle dog.

3. The sustained-release injectable composition containing dutasteride according to claim 2, wherein the maximum plasma concentration (Cₘₐₓ) of the dutasteride is 200 ng/mL to 2,500 ng/mL.

4. The sustained-release injectable composition containing dutasteride according to claim 1, wherein the microparticles comprise two or more biodegradable polymers selected from the group consisting of polylactic acid, polylactide, polylactide-co-glycolide (PLGA), polyphosphazine, polyiminocarbonate, polyphosphoester, polyanhydride, polyorthoester, polycaprolactone, polyhydroxyvalerate, polyhydroxybutyrate, and polyamino acids.

5. The sustained-release injectable composition containing dutasteride according to claim 4, wherein the biodegradable polymers comprise polylactic acid and polylactide-co-glycolide (PLGA) at a weight ratio of 1:1 to 1:4.

6. The sustained-release injectable composition containing dutasteride according to claim 1, wherein the microparticles comprise the dutasteride and the biodegradable polymers at a weight ratio of 1:2 to 1:5.

7. The sustained-release injectable composition containing dutasteride according to claim 1, wherein the microparticles release less than 50 wt% of the dutasteride for 24 hours, as a result of conducting an accelerated release test under the following conditions:
[Test conditions]
Water containing 1% sodium lauryl sulfate is used as a dissolution test solution, the microparticles are mixed with the dissolution test solution, a shaking water bath is used as a dissolution tester, a glass test container with a capacity of 120 ml is used as a dissolution test container, and shaking is performed at a speed of 120 rpm at 45°C.

8. The sustained-release injectable composition containing dutasteride according to claim 1, wherein the microparticles further comprise a coating layer on outer surfaces thereof.

9. The sustained-release injectable composition containing dutasteride according to claim 1, wherein a standard deviation (SD) for diameters of the microparticles is 2 to 7.

10. The sustained-release injectable composition containing dutasteride according to claim 1, wherein a width of a peak in results of particle size analysis (PSA) of the microparticles is 5 to 15.
